# EUROPEAN PATENT SPECIFICATION

(11) **EP 4 357 064 B1**
(45) Date of publication and mention of the grant of the patent: **15.10.2025**
(21) Application number: 22202430.9
(22) Date of filing: 19.10.2022
(51) Int. Cl.: B23K 26/16, B23K 26/38, A61F 2/91

(54) **LASER CUTTING SYSTEM AND BEAM BLOCK FOR SUCH SYSTEM**
LASERSCHNEIDSYSTEM UND STRAHLFÄNGER FÜR EIN SOLCHES SYSTEM
SYSTÈME DE DÉCOUPE AU LASER ET ARRETEUR DE FAISCEAU POUR UN TEL SYSTÈME

(43) Date of publication of application: 24.04.2024
(73) Proprietor: BIOTRONIK AG, 8180 Bülach (CH)
(72) Inventor: Neckin, André, 18059 Rostock (DE); Klemm, Ralf, 18209 Parkentin (DE); Burgschat, André, 18057 Rostock (DE); Graentzel, Ole, 18106 Rostock (DE); Schacht, Gilbert, 18059 Rostock (DE)
(74) Representative: WSL Patentanwälte Partnerschaft mbB

(56) References cited:
- WO-A1-2022/093537
- US-A1- 2004 089 643

## Description

The invention is directed to a beam block for laser cutting to produce a perforated tubular workpiece, for example a stent or a stent structure, from a tubular semi-finished product, an operation method of such beam block, a laser cutting system comprising such beam block, a manufacturing method for such perforated tubular workpiece using such laser cutting system, and a workpiece manufactured according to such manufacturing method.

Small perforated tubular workpieces such as stents or stent structures are often produced by laser cutting from a tubular semi-finished product, which may be a full or a partly full tube. A stent or a stent structure is a perforated metal or plastic tube that may be inserted (implanted) into the lumen of an anatomic vessel or duct of a human being or an animal to keep the passageway open or to fix an implant at the inner wall of the vessel such as an artificial valve. There is a wide variety of stents or stent structures used for different purposes, for example, mechanical stents or stent structures, balloon-expandable stents, and/or self-expanding coronary or peripheral stents, vascular and biliary stents, stent grafts or covered stents (covered with fabric or biological material, for example, cellulose, bacterial cellulose or biological tissue, for example, pericardium or treated pericardium), prosthetic heart valve stents, ureteral stents, prostatic stents, colon and esophageal stents, pancreatic and biliary stents, and glaucoma drainage stents and occluder, for example LAA-occluder.

During laser processing of such perforated tubular workpiece the laser cuts through the material of the semi-finished product thereby producing small structures having a diameter of, for example, less than a tenth of a millimeter such as holes, struts, webs, wires and web/strut connection, i.e. the perforated structure. Nowadays, a laser cutting system for such small structures uses a microsecond pulsed laser that work according to a melt cutting process (also referred to as fusion cutting process). In such process the material is heated to its melting point and a gas jet blows the molten material out of the kerf avoiding the raise the temperature of the material any further and avoiding oxidation at the area of the cut. As the semi-finished product is a tube or a partly full tube, a beam block is accommodated within the inner cavity of the tube to focus laser machining to the one of the opposing tube walls that is closest to the laser nozzle and avoid undesirable laser process induced material alterations of the respective opposite tube wall.

A beam block located within the workpiece may have the form of a cylinder, a tube or a section of such cylinder or tube. The above-described melt and blow process of molten material causes deposition of molten material at the surface of such beam block that may accumulate and attach to the surface of the beam block so that the gas jet cannot or cannot fully remove such materials accumulation. The accumulated material may contact the inner surface of the workpiece during laser processing thereby causing dimensional deviations and/or defects at the inner surface of the workpiece and workpiece rejects.

US 2004/089643 A1 relates to an improved expandable stent for implantation in a body lumen, such as an artery, and an improved method for making it from a single length of tubing. The stent is manufactured by direct laser cutting from a single metal tube using a finely focused laser beam originating from a diode pumped fiber laser with an external pulse generator and passing through a coaxial gas jet structure to impinge on the working surface of the tube as the linear and rotary velocity of the tube is precisely controlled. To optimize the cut, the laser parameters may be adjusted and/or the laser pulse may be shaped.

WO 2022/093537 A1 relates to an apparatus, and associated method, laser cuts self-wrapping, woven or braided split sleeves from a continuous feed of sleeve material by sweeping a laser beam across the continuously fed material. Instead of sweeping the laser beam straight across the material in a direction perpendicular to the longitudinal axis of the material, the sweep path is angled to follow the feed rate of the mate1ial while the laser beam cuts through the material from one side to the other. Thus, a straight cut may be completed without stopping the feed. The apparatus includes a mandrel tor expanding the material before intersection with the laser beam to open a gap at the longitudinal split. The mandrel has a wedge-shaped tip with an end-surface profile that is at an oblique angle to the direction of motion of the material. The oblique angle at least approximately matches the sweep angle of the laser beam.

Consequently, it is desirable to provide a beam block, a laser cutting system and respective operation and manufacturing methods for reduction or avoidance of dimensional deviations and defects of perforated tubular workpieces.

The above problem is solved by a beam block with the features of claim 1, a laser cutting system with the features of claim 7, an operation method of a beam block with the features of claim 9 and a manufacturing method for a perforated tubular workpiece with the features of claim 13.

In particular, the above problem is solved by a beam block for laser cutting to produce a perforated tubular workpiece, for example a stent or a stent structure, from a tubular semi-finished product, wherein the beam block comprises
- a tubular head section configured to be accommodated within a semi-finished product inner cavity during laser cutting, wherein the tubular head section has a recess at its first end that allows depositing molten workpiece material at an inner surface of the head section,
- a shaft section located at a second end of the tubular head section, and
- a plunger element configured to be reciprocated along a head section inner cavity to the first end of the tubular head section thereby ejecting molten workpiece material deposited at an inner surface of the head section.

The above beam block is used for laser cutting a tube or a partly full tube of a semi-finished product by a melt cutting process as indicated above using a microsecond pulsed laser. Preferably, the microsecond pulsed laser has an emission wavelength in the Near-Infrared, for example, within the range of 1000 to 1100 nm. Such laser may be, for example, a fiber laser emitting in the Near-Infrared. The above beam block is used to manufacture laser cut perforated tubular workpieces such as stents or stent structures, e.g. expandable coronary, vascular and biliary stents, stent grafts or covered stents (covered with fabric), ureteral stents, prostatic stents, colon and esophageal stents, pancreatic and biliary stents, and glaucoma drainage stents.

The beam block comprises a head section and a shaft section, wherein the shaft section is fixed to the head section at the second end of the head section. The head section forms the seat for the tubular semi-finished product during laser cutting, i.e. the semi-finished product located at the tubular head section of the beam block in the area of the first end and the recess. The tubular head section is slid into the inner cavity of the semi-finished product prior laser cutting. During the laser cutting process the tube wall closest to the laser and its nozzle is cut, the beam block blocks the laser beam and thereby avoids that the laser produces material alterations at the opposite tube wall. The recess runs at least partly inclined with regard to the longitudinal axis of the tubular head section and is provided such that a part of the head section wall is removed, wherein the removed part is enlarged into the direction of the first end of the head section. Due to the removal of a part of the head section wall, the gas jet of the laser intrudes into the inner cavity of the head section of the beam block. Accordingly, during laser cutting the gas jet transports molten material of the workpiece removed by the laser and the gas jet. This molten material accumulates at the inner surface of the inner cavity of the head section.

According to the invention this molten material is removed by the plunger element that reciprocates to the first end along the head section inner cavity. For example, the initial position of the leading end (first end) of the plunger element is at the second end of the beam block head section, so that it reciprocates from the second end to the first end along the head section inner cavity.

The invention provides a cost-effective, fast and easy to realize removal of the molten material thereby considerably reducing the reject of perforated tubular workpieces. A test production of laser cut stents using the above described beam block revealed a reduction to about one third of rejected stents. Accordingly, as less workpieces need to be sorted out due to incorrect dimensions, material expenses as well as laser cutter running time are reduced. This leads to reduction of personnel costs and quality check costs, as well.

In one embodiment, the plunger element has a simple and easy to realize wire-like or cylindrical form, at least at its leading end (first end), that moves with little play within the inner cavity of the head section of the beam block. In one embodiment, the plunger element has a wire-like form along its full length. The outer diameter of the plunger element leading end reaching the very first end of the head section during reciprocating movement is provided such that it is little less than the inner diameter of the inner cavity of the head section of the beam block. In one embodiment, the plunger element has this outer diameter along its full length. During reciprocating movement the plunger element moves forward to the first end (i.e. during the forward movement phase of the reciprocating movement), thereby it scrapes off the molten workpiece material accumulated at the inner surface of the head section inner cavity and pushes the molten workpiece material to the very first end of the head section so that it falls out of the head section inner cavity into a collecting box or into a ready cut section of the tubular semi-finished product which overhangs the very first end of the head section, and from there into the collecting box. In the collecting box the molten material is collected and may be recycled lateron. Then, the plunger element moves backward (i.e. during the backward movement phase of the reciprocating movement) from the first end back to its initial position, e.g. to the second end of the beam block head section. The plunger element may have a length that is about the length of the beam block head section and shaft section or greater. The realization of the reciprocating movement of the plunger element is cost-effective and easy to control.

The plunger element may comprise or consist of a metal material, for example V2A steel. The beam block head section and/or shaft section may comprise or consist of a metal material, for example a thermally stable alloy. These materials effectively block the laser radiation and reliably provide the scraping and pushing function with regard to the molten material collecting at the inner cavity of the beam block head section, respectively.

As indicated above, in one embodiment of the beam block, the plunger element has a wire-like form and/or is connected to and driven by a pneumatic, electric or hydraulic driving unit, wherein the driving unit comprises, for example, a pneumatic cylinder, e.g. a double acting pneumatic cylinder. The plunger element may be connected to the driving unit, for example, by a web-like element. Such driving unit reliably moves the plunger element and is easy controllable.

In one embodiment of the beam block, the shaft section has a tubular form and/or the plunger element reciprocates along the shaft section. In one embodiment, the head section is fixed to the shaft section by an adhesive joint. For example, the adhesive joint of the head section is formed by a section of the outer shell surface at the second end of the head section to which an inner surface of the inner cavity of the shaft section is fixed. Alternatively, vice versa an outer shell surface of the shaft section is fixed to an inner surface of the inner cavity of the head section of the beam block. In an alternative embodiment, the head section may be fixed to the shaft section by a positive and/or friction locking connection. It is important, that the inner cavities of the head section and the shaft section form a continuous cavity in which the plunger element extending along the head and shaft section may smoothly reciprocate. As indicated above, the plunger element length may greater than the longitudinal length of the head and shaft section of the beam block. It extends from the end of the shaft section of the beam block where it is connected to the driving unit. The above-described construction of the beam block provides reliable reciprocating movement of the plunger element within the inner cavity of the shaft and head section of the beam block due to a correct guidance.

In one embodiment, the beam block comprises a tubular sleeve accommodated at a part of the shell surface of the head section of the beam block having a non-stick shell surface. In one embodiment, the tubular sleeve comprises or consist of Polytetrafluoroethylene (PTFE) forming the non-stick shell surface. The tubular sleeve is fixed to the outer shell surface of the head section by an adhesive joint. The tubular sleeve smoothens the gliding/sliding of the beam block along a guiding surface, for example during introduction into the semi-finished product prior lasering. The beam block may comprise two or more of such tubular sleeves along its entire length, along its head and shaft section. Additionally or alternatively, there may be a PTFE block comprising a through-going opening as a guide for the shaft section of the beam block which also provides a reliable non-sticking guidance of the beam block.

In one embodiment of the beam block, the driving unit comprises a damping component configured to damp the plunger element movement at the end of at least one of the forward movement phase and the backward movement phase of the reciprocating movement. For example, the damping may be realized by a throttle check valve. The damping avoids transfer of vibrations caused by the plunger element movement to the semi-finished product thereby preventing accuracy reduction of laser cutting and increases the operation lifetime of the pneumatic cylinder.

Further, the above problem is solved by a laser cutting system comprising the above-described beam block and a laser cutter, wherein the head section of the beam block is configured to be accommodated within a tubular semi-finished product to be cut by the laser cutter, wherein a nozzle of the laser cutter is located within a pre-defined radial distance from an outer shell surface of the semi-finished product. The laser cutter may comprise a laser providing µs pulses with an excitation wavelength in the Near-Infrared, for example in the range between 1000 nm to 1100 nm. The high pressure process gas for cutting may be, for example, argon. In one embodiment, the cutting velocity may be 20 mm/s or less. The radial distance of the laser cutter from the surface of the semi-finished product may be, for example, in between one and five tenths of a millimeter. Similar to the beam block, the laser cutting system reduces productions costs for perforated tubular workpieces.

Further, the above problem is solved by an operation method of above-described beam block, wherein the plunger element is reciprocated along the head section inner cavity to the first end of the tubular head section thereby ejecting molten workpiece material deposited at an inner surface of the head section. As indicated above, the plunger element moves in a forward movement phase to the very first end of the head section of the beam block thereby scraping the deposited molten material from the inner surface of the head section, pushing it to the first end of the head section so that it falls out of the inner cavity. The above operation method realizes a method that is cost effective also because it does not need any additional time as it may be used during laser cutting of the semi-finished product.

In one embodiment of the operation method, the plunger element is periodically reciprocated having a frequency in the range of 0,2 min⁻¹ to 0,0055 Hz. This frequency was proven to remove the accumulated molten material during laser cutting in a sufficient way during test production. Depending on the speed of the laser cutting and of the thickness of the wall of the tubular semi-finished product the frequency may be even lower or higher than 0,2 min⁻¹ to 0,0055 Hz. The same applies to the embodiment of the operation method, wherein the duration of one reciprocating movement of the plunger element is in the range of 0.5 s to 10 s, preferably in the range of 0.5 s to 1.5 s.

In one embodiment of the operation method, the reciprocating movement of the plunger element is damped at the end of at least one of the forward movement phase and the backward movement phase of the reciprocating movement. As indicated above, the damping reduces vibrations that may otherwise be transferred to the semi-finished product during lasering and may interfere with laser cutting.

Furthermore, the above problem is solved by a manufacturing method for a perforated tubular workpiece from a tubular semi-finished product using the above-described laser cutting system and comprising the following steps:
- accommodating the beam block within the semi-finished product such that the product is located within the area of the first end of the tubular head section,
- laser cutting the semi-finished product using the laser cutter, and
- operating the beam block according to the above-described operation method.

In one embodiment, the laser cutting is provided by the laser as described above, for example using the melt cutting process. The laser cuts perforations such that structures are formed in wall of the tubular semi-finished product, wherein the perforations are cut through the whole wand thickness of the semi-finished product. The structures are, for example, holes, struts, webs, wires and web/strut connections or similar structures comprising any combination of these structures. In one embodiment, the plunger element reciprocating movement of the beam block operation is provided periodically during laser cutting the semi-finished product. I.e. the beam block, in particular the plunger element, is operated, for example, during laser cutting of the semi-finished product with a frequency as indicated above. Each molten material removal step, i.e. each reciprocated movement has the duration as disclosed above. The manufacturing method comprises the above advantages of the beam block and its operation method as well as of the laser cutting system.

The various features and advantages of the present invention may be more readily understood with reference to the following detailed description and the embodiments shown in the drawings. Herein schematically and exemplarily,
- Fig. 1 to 3: depict a first embodiment of a laser cutting system with a first embodiment of a beam block in a side view in three different operation states,
- Fig. 4: shows a second embodiment of a beam block in a side view,
- Fig. 5: depicts the embodiment of Fig. 4 in a longitudinal section along the arrows A-A shown in Fig. 4,
- Fig. 6: shows the head section of the embodiment of Fig. 4 in a top view, and
- Fig. 7: depicts the head section and a part of the shaft section of the embodiment of Fig. 4 in a perspective view of a longitudinal section.

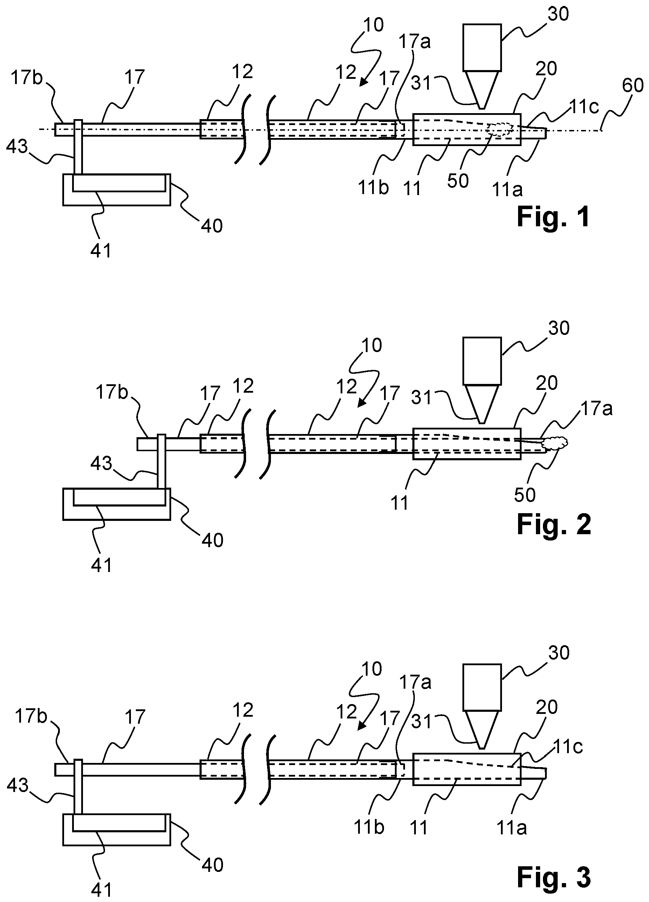

The laser cutting system shown in Fig. 1 to 3 comprises a beam block 10 and a semi-finished tubular product 20 which is cut by a laser 30 thereby producing a perforated tubular workpiece such as a stent. The beam block 10 comprises a tubular head section 11 and a tubular shaft section 12, wherein the head section 11 is fixed to the shaft section 12 at its second end 11b. The head section 11 and the shaft section 12 of the beam block 10 extend along a longitudinal axis 60 and form a common inner cavity which accommodates a plunger element 17. One end of the beam block 10 is formed by the first end 11a of the head section 11. The head section 11 of the beam block 10 is located within the inner cavity of the semi-finished product 20 during lasering as shown in Fig. 1 to 3. The laser 30, for example a µs-fiber laser, providing µs pulses, cuts through the wall of the semi-finished product 20 consisting, for example, of Nitinol using a melt cutting process. As a process gas, for example, argon is used producing the jet stream. The laser beam and the jet stream are directed onto the outer shell surface of the semi-finished product 20 from a radial direction (referred to the longitudinal direction represented by the longitudinal axis 60) for laser cutting, wherein the front end of a nozzle 31 of the laser cutter 30 has a pre-defined radial distance from the outer shell surface of the semi-finished product. By cutting perforations, structures like holes, webs, struts, wires or similar structures or any combination of such structures are formed in the wall of the semi-finished product.

The head section 11 further comprises a recess 11c that is located at the first end 11a of the head section and extends from there into the direction of the second end 11b. The recess 11c is formed partly inclined and such that the wall of the tube of the head section 11 is removed on the upper side (if one refers to the view shown in Fig. 1 to 3). The inclination is such that the removed part of the tube wall is greater at the first end 11a of the head section 11 than at the opposite end of the recess 11c (with regard to the longitudinal direction). As the tube wall is removed along the extension of the recess 11c the inner cavity of the tubular head section 11 is exposed and molten material 50 accumulates within the cavity as shown in Fig. 1.

The opposite end of the beam block 11 along the longitudinal direction represented by longitudinal axis 60 is formed by the second end 17b of the plunger element 17. The plunger element 17 further has a leading end (first end 17a) that is opposite to the second end 17b along the longitudinal direction. The plunger element 17 may consist of V2A steel, for example. In the initial state/position, the plunger element 17 extends from the second end 11b of the head section 11 to the end of the shaft section 12 opposite the head section 11 and further the plunger element 17 projects from the shaft section 12 of the beam block 10. There, at the second end 17b, the plunger element 17 is connected to a driving unit 40. In particular, the plunger element 17 is connected to a double acting pneumatic cylinder 41 of the driving unit 40 by a stiff arm 43. The pneumatic cylinder 41 drives the plunger element 17 via the arm 43 from an initial state/position shown in Fig. 1 into the direction of the first end 11a of the head section 11 and back into the initial position as shown in Fig. 2 and 3, respectively, i.e. the plunger element 17 performs a reciprocating movement driven by the pneumatic cylinder 41.

Fig. 1 shows the initial state/position of the plunger element 17 in which the plunger element is retracted within the common inner cavity of the head section 11 and the shaft section 12 of the beam block. Driven by the pneumatic cylinder 41 of the driving unit 40 the front end 17a of the plunger element 17 is moved during a forward phase of the reciprocating movement along the longitudinal direction to the first end 11a of the head section 11 thereby scraping off the inner surface of the head section 11 and pushing the molten and accumulated material 50 of the semi-finished product 20 to and over the first end 11a of the head section 11 into a container (not shown) that collects the molten material 50. Then, during the backward phase of the reciprocating movement the plunger element 17 is driven by the pneumatic cylinder 41 into the opposite direction along the longitudinal axis 60, wherein at the end of the movement the plunger element 17 takes the initial position/state as shown in Fig. 3. The molten material 50 is removed. The duration of one reciprocating movement of the plunger element 17 (or the pneumatic cylinder 41) is, for example, between 1 s and 10 s. The reciprocating movement may be periodically repeated with a frequency, for example, in the range of 0,2 min⁻¹ to 0,0055 Hz.

At the end of the forward phase and the backward phase the movement of the pneumatic cylinder 41 and thereby the movement of the plunger element 17 may be damped, for example by a throttle check valve

For manufacturing of a perforated tubular workpiece, e.g., a stent, at first the laser cutting system as described above and a semi-finished product 20 for such stent is provided. Then, at first the head section 11 of the beam block 10 is accommodated within the inner cavity of the semi-finished product 20. After correct positioning of the beam block 10 within the semi-finished product and the laser cutter 30 in relation to the semi-finished product 20, laser cutting starts using the laser cutter 30. During laser cutting the plunger element is periodically activated to provide the reciprocating movement thereby removing the molten material 50 accumulated within the inner cavity of the head section 11 of the beam block 10. During laser cutting, the laser cutter 30 may be moved relative to the outer shell surface of the semi-finished product 20.

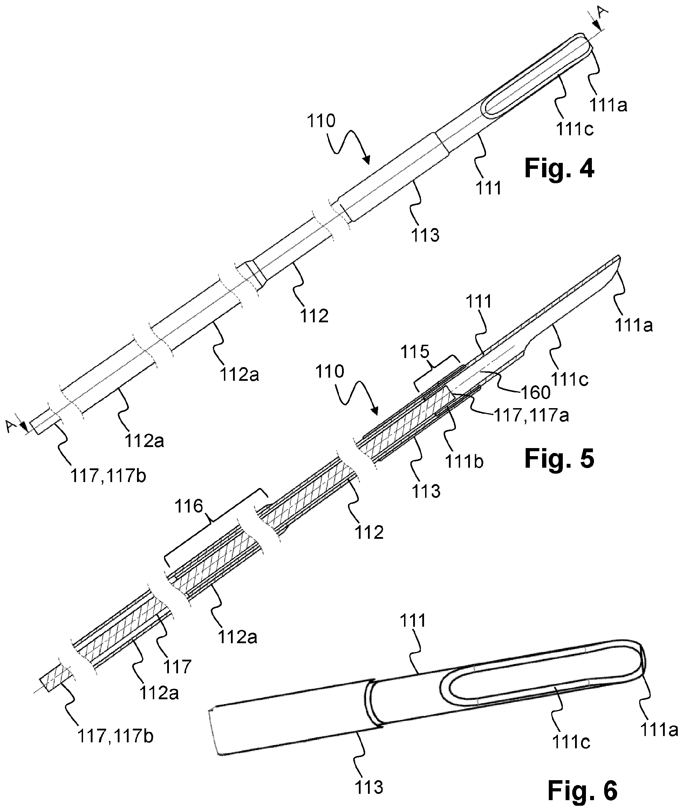

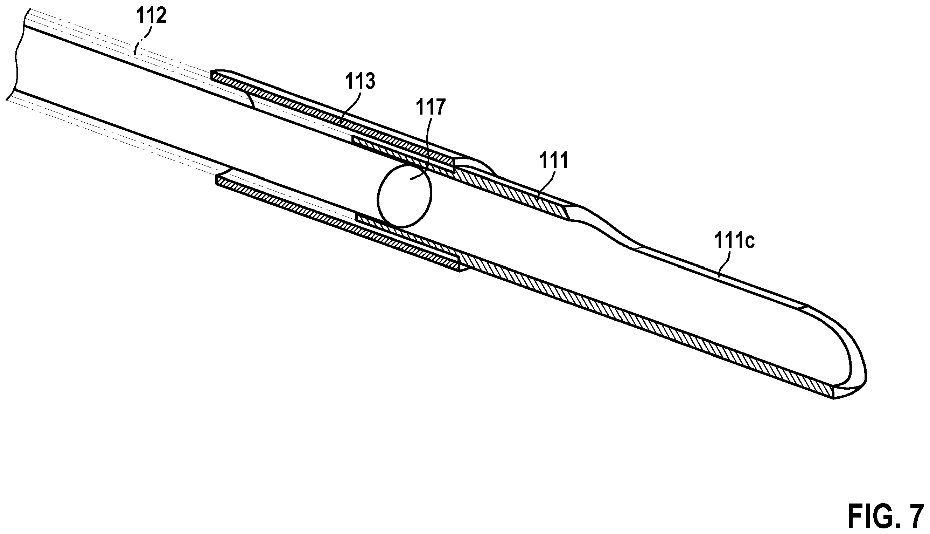

The second embodiment of a beam block 110 depicted in Fig. 4 to 7 basically corresponds to the beam block 10 of the first embodiment. Accordingly, the reference numbers of the second embodiment minus the number 100 correspond to the reference numbers of similar elements of the beam block of the first embodiment. With regard to these elements it is referred to the first embodiment. The driving unit and the arm connecting the plunger element 117 to the driving unit are not shown but similar to the first embodiment. However, one difference is that the shaft section of the beam block 110 comprises two separate tubular parts, a first shaft section 112 and a second shaft section 112a. The first and the second shaft sections 112, 112a are fixed to each other by an adhesive connection within the section 116 of the beam block 110. The second shaft section 112a which is located further away from the head section 111 than the first shaft section 112 has a greater inner and outer diameter than the first shaft section 112.

As one can see more clearly from the second embodiment, the inner cavity of the head section 111 and the shaft section 112, 112a is a common cavity which has a slightly larger diameter than the outer diameter of the plunger element 117 so that the plunger element is allowed to reciprocate within this common cavity for removal of the molten material.

Further, the second embodiment of the beam block 110 comprises a tubular sleeve 113 which is accommodated at the outer shell surface of head section 111 and the first shaft section 112 in the area where the head section 111 and the first shaft section are fixed to each other by an adhesive connection. This tubular sleeve 113 is fixed to the outer surface of the head section 111 and the first shaft section 112 by an adhesive connection, as well. The tubular sleeve 113 consists of, for example, PTFE thereby providing a non-stick shell surface. This allows easy sliding of the whole beam block 110 within a holding groove, for example, during introduction within the semi-finished product. In one embodiment, the beam block 110 may comprise not only the one tubular sleeve with the non-stick shell surface shown in Fig. 4 and 5 but two or more of such sleeves along the full length of the beam block.

Additionally, the second embodiment shows one example of the recess 111c at the first end 111a of the head section 111 of the beam block 110. It is clearly shown in Fig. 4 to 7 that at the recess 111c the wall of the tubular head section 111 is removed on one side thereby exposing the inner cavity of the head section 111 for accumulation of the molten material. In the center section, the edge of the recess 111c runs approximately parallel to the longitudinal direction, whereas at the first and second end of the recess 111c the edges are inclined with regard to the longitudinal direction.

As indicated above, the removal of the molten workpiece material by the plunger element of the beam block during laser cutting considerably reduces production costs as the perforated workpiece reject rate is significantly decreased.

## Claims

1. A beam block (10, 110) for laser cutting to produce a perforated tubular workpiece, for example a stent, from a tubular semi-finished product (20), wherein the beam block (10, 110) comprises
• a tubular head section (11) configured to be accommodated within a semi-finished product inner cavity during laser cutting, wherein the tubular head section (11, 111) has a recess (11c, 111c) at its first end (11a, 111a) that allows depositing molten workpiece material (50) at an inner surface of the tubular head section (11, 111),
• a shaft section (12, 112, 112a) located at a second end (11b, 111b) of the tubular head section (11, 111), and **characterized in that** the beam block further comprises
• a plunger element (17, 117) configured to be reciprocated along a tubular head section inner cavity to the first end (11a, 111a) of the tubular head section (11, 111) thereby ejecting molten workpiece material (50) deposited at an inner surface of the tubular head section (11, 111).

2. The beam block of claim 1, wherein the plunger element (17, 117) has a wire-like form and/or is connected to and driven by a pneumatic, electric or hydraulic driving unit (40), wherein the driving unit (40) comprises, for example, a pneumatic cylinder (41), e.g. a double acting pneumatic cylinder.

3. The beam block of any one of the previous claims, wherein the shaft section (12, 112, 112a) has a tubular form and/or the plunger element (17, 117) reciprocates along the shaft section.

4. The beam block of any one of the previous claims, wherein the beam block (10, 110) comprises a tubular sleeve (113) accommodated at a part of the shell surface of the head section (11, 111) of the beam block having a non-stick shell surface.

5. The beam block of any one of the previous claims, wherein the head section (11, 111) is fixed to the shaft section (12, 112, 112a) by an adhesive joint.

6. The beam block of any one of the previous claims, wherein the driving unit (40) comprises a damping component configured to damp the plunger element movement at the end of at least one of the forward movement phase and the backward movement phase of the reciprocating movement.

7. A laser cutting system comprising the beam block (10, 110) of any one of the previous claims and a laser cutter (30), wherein the head section (11, 111) of the beam block is configured to be accommodated within a tubular semi-finished product (20) to be cut by the laser cutter (30), wherein a nozzle (31) of the laser cutter (30) is located within a pre-defined radial distance from an outer shell surface of the semi-finished product (20).

8. The laser cutting system of claim 7, wherein the laser cutter (30) comprises a laser providing µs pulses and using, for example, argon as a high pressure gas.

9. An operation method of a beam block of any one of the claims 1 to 6, wherein the plunger element (17, 117) is reciprocated along the head section inner cavity to the first end (11a, 111a) of the tubular head section (11, 111) thereby ejecting molten workpiece material (50) deposited at an inner surface of the head section (11, 111).

10. The operation method according to claim 9, wherein the plunger element (17, 117) is periodically reciprocated having a frequency in the range of 0,2 min⁻¹ to 0,0055 Hz.

11. The operation method according to any one of the claims 9 to 10, wherein the duration of one reciprocating movement of the plunger element (17, 117) is in the range of 0.5 s to 10 s, preferably in the range of 0.5 s to 1.5 s.

12. The operation method according to any one of the claims 9 to 11, wherein the reciprocating movement of the plunger element (17, 117) is damped at the end of at least one of the forward movement phase and the backward movement phase of the reciprocating movement.

13. A manufacturing method for a perforated tubular workpiece from a tubular semi-finished product (20) using the laser cutting system of any one of the claims 7 to 8 and comprising the following steps:
• accommodating the beam block (10, 110) within the semi-finished product such that the product (20) is located within the area of the first end (11a, 111a) of the tubular head section (11, 111),
• laser cutting the semi-finished product using the laser cutter (30), and
• operating the beam block (10, 110) according to the operation method of any one of the claims 9 to 11.

14. The manufacturing method according to claim 13, wherein the plunger element reciprocating movement of the beam block operation is provided periodically during laser cutting of the semi-finished product (20).

## Patentansprüche

1. Strahlfänger (10, 110) für das Laserschneiden zur Herstellung eines perforierten rohrförmigen Werkstücks, beispielsweise eines Stents, aus einem rohrförmigen Halbzeug (20), wobei der Strahlfänger (10, 110) aufweist
• einen rohrförmigen Kopfabschnitt (11), der derart konfiguriert ist, dass er während des Laserschneidens in einem Innenhohlraum des Halbzeugs aufgenommen werden kann, wobei der rohrförmige Kopfabschnitt (11, 111) an seinem ersten Ende (11a, 111a) eine Ausnehmung (11c, 111c) hat, die ein Ablagern von geschmolzenem Werkstückmaterial (50) an einer Innenfläche des rohrförmigen Kopfabschnitts (11, 111) ermöglicht,
• einen Schaftabschnitt (12, 112, 112a), der an einem zweiten Ende (11b, 111b) des rohrförmigen Kopfabschnitts (11, 111) angeordnet ist, und **dadurch gekennzeichnet, dass** der Strahlfänger ferner aufweist
• ein Stößelelement (17, 117), das derart konfiguriert ist, dass es entlang eines Hohlraums des rohrförmigen Kopfabschnitts zu dem ersten Ende (11a, 111a) des rohrförmigen Kopfabschnitts (11, 111) hin- und herbewegt werden kann, wodurch an einer Innenfläche des rohrförmigen Kopfabschnitts (11, 111) abgelagertes geschmolzenes Werkstückmaterial (50) herausgebracht wird.

2. Strahlfänger nach Anspruch 1, wobei das Stößelelement (17, 117) eine drahtartige Form aufweist und/oder mit einer pneumatischen, elektrischen oder hydraulischen Antriebseinheit (40) verbunden ist und von dieser angetrieben wird, wobei die Antriebseinheit (40) beispielsweise einen Pneumatikzylinder (41), z. B. einen doppeltwirkenden Pneumatikzylinder, aufweist.

3. Strahlfänger nach einem der vorstehenden Ansprüche, wobei der Schaftabschnitt (12, 112, 112a) eine rohrförmige Form aufweist und/oder das sich Stößelelement (17, 117) entlang des Schaftabschnitts hin- und herbewegt.

4. Strahlfänger nach einem der vorstehenden Ansprüche, wobei der Strahlfänger (10, 110) eine rohrförmige Hülse (113) aufweist, die an einem Teil der Mantelfläche des Kopfabschnitts (11, 111) des Strahlfängers mit einer nicht haftenden Mantelfläche aufgenommen ist.

5. Strahlfänger nach einem der vorstehenden Ansprüche, wobei der Kopfabschnitt (11, 111) durch eine Klebeverbindung an dem Schaftabschnitt (12, 112, 112a) befestigt ist.

6. Strahlfänger nach einem der vorstehenden Ansprüche, wobei die Antriebseinheit (40) ein Dämpfungselement aufweist, das derart konfiguriert ist, dass es die Bewegung des Stößelelements am Ende mindestens einer der Vorwärtsbewegungsphasen und der Rückwärtsbewegungsphasen der Hin- und Herbewegung dämpft.

7. Laserschneidsystem, das den Strahlfänger (10, 110) nach einem der vorstehenden Ansprüche und einen Laserschneider umfasst (30), wobei der Kopfabschnitt (11, 111) des Strahfängers derart konfiguriert ist, dass er in einem rohrförmigen Halbzeug (20) aufgenommen werden kann, das durch den Laserschneider (30) geschnitten werden soll, wobei eine Düse (31) des Laserschneiders (30) in einem vordefinierten radialen Abstand von einer Außenmantelfläche des Halbzeugs (20) angeordnet ist.

8. Laserschneidsystem nach Anspruch 7, wobei der Laserschneider (30) einen Laser aufweist, der µs-Pulse liefert und beispielsweise Argon als Hochdruckgas verwendet.

9. Verfahren zum Betätigen eines Strahlfängers nach einem der Ansprüche 1 bis 6, wobei das Stößelelement (17, 117) entlang des inneren Hohlraums des Kopfabschnitts zum ersten Ende (11a, 111a) des rohrförmigen Kopfabschnitts (11, 111) hin- und herbewegt wird, wodurch geschmolzenes Werkstückmaterial (50), das sich an einer Innenfläche des Kopfabschnitts (11, 111) abgelagert hat, herausgebracht wird.

10. Verfahren nach Anspruch 9, wobei das Stößelelement (17, 117) periodisch mit einer Frequenz im Bereich von 0,2 min⁻¹ bis 0,0055 Hz hin- und herbewegt wird.

11. Verfahren nach einem der Ansprüche 9 bis 10, wobei die Dauer einer Hin- und Herbewegung des Stößelelements (17, 117) im Bereich von 0,5 s bis 10 s, vorzugsweise im Bereich von 0,5 s bis 1,5 s liegt.

12. Verfahren nach einem der Ansprüche 9 bis 11, wobei die Hin- und Herbewegung des Stößelelements (17, 117) am Ende mindestens einer der Vorwärtsbewegungsphase und der Rückwärtsbewegungsphase der Hin- und Herbewegung gedämpft wird.

13. Verfahren zur Herstellung eines perforierten rohrförmigen Werkstücks aus einem rohrförmigen Halbzeug (20) unter Verwendung des Laserschneidsystems nach einem der Ansprüche 7 bis 8, das die folgenden Schritte aufweist:
• Aufnehmen des Strahlfängers (10, 110) in das Halbzeug, so dass das Produkt (20) innerhalb des Bereichs des ersten Endes (11a, 111a) des rohrförmigen Kopfabschnitts (11, 111) angeordnet ist,
• Laserschneiden des Halbzeugs mit dem Laserschneider (30) und
• Betätigen des Strahlfängers (10, 110) gemäß dem Verfahren zum Betätigen nach einem der Ansprüche 9 bis 11.

14. Herstellungsverfahren nach Anspruch 13, wobei die Stößelelement-Hin- und Herbewegung des Strahlfänger-Betriebs periodisch während des Laserschneidens des Halbzeugs (20) vorgesehen ist.

## Revendications

1. Bloc à faisceau (10, 110) pour une découpe au laser pour produire une pièce de travail tubulaire perforée, par exemple un stent, à partir d'un produit semi-fini (20) tubulaire, le bloc à faisceau (10, 110) comprenant
• une partie de tête tubulaire (11) configurée pour être logée dans une cavité intérieure d'un produit semi-fini pendant la découpe au laser, la partie de tête tubulaire (11, 111) ayant un renfoncement (11c, 111c) à sa première extrémité (11a, 111a) qui permet de déposer du matériau de travail (50) fondu sur une surface intérieure de la partie de tête tubulaire (11, 111),
• une partie de tige (12, 112, 112a) située à une deuxième extrémité (11b, 111b) de la partie de tête tubulaire (11, 111), et
**caractérisé en ce que** le bloc à faisceau comprend en outre
• un élément piston (17, 117) configuré pour être déplacé en va-et-vient le long d'une cavité intérieure de la partie de tête tubulaire vers la première extrémité (11a, 111a) de la partie de tête tubulaire (11, 111) en éjectant par cela du matériau de travail (50) fondu déposé sur une surface intérieure de la partie de tête tubulaire (11, 111).

2. Bloc à faisceau selon la revendication 1, l'élément piston (17, 117) ayant une forme de fil de fer et/ou étant connecté à et étant entraîné par une unité d'entraînement pneumatique, électrique ou hydraulique (40), l'unité d'entraînement (40) comprenant, par exemple, un cylindre pneumatique (41), par exemple un cylindre pneumatique à double effet.

3. Bloc à faisceau selon l'une des revendications précédentes, la partie de tige (12, 112, 112a) ayant une forme tubulaire et/ou l'élément piston (17, 117) des déplaçant en va-et-vient le long de la partie de tige.

4. Bloc à faisceau selon l'une des revendications précédentes, le block à faisceau (10, 110) comprenant un manchon tubulaire (113) agencé à une partie de la surface enveloppante de la partie de tête (11, 111) du block à faisceau ayant une surface enveloppante non collante.

5. Bloc à faisceau selon l'une des revendications précédentes, la partie de tête (11, 111) étant fixée à la partie de tige (12, 112, 112a) par un joint adhésif.

6. Bloc à faisceau selon l'une des revendications précédentes, l'unité d'entraînement (40) comprenant un composant amortissant configuré pour amortir le mouvement de l'élément piston à la fin d'au moins un des mouvements avant et arrière du mouvement de va-et-vient.

7. Système de découpe au laser comprenant le bloc à faisceau (10, 110) selon l'une des revendications précédentes, et un couteau laser (30), la partie de tête (11, 111) du block à faisceau étant configuré pour être logé à l'intérieur d'un produit semi-fini (20) tubulaire à couper par le couteau laser (30), un bec (31) du couteau laser (30) étant disposé à une distance radiale prédéfinie d'une surface enveloppante extérieur du produit semi-fini (20).

8. Système de découpe selon la revendication 7, le couteau laser (30) comprenant un laser fournissant des impulsions laser µs et utilisant, par exemple, de l'argon comme gaz à haute pression.

9. Procédé de fonctionnement d'un bloc à faisceau selon l'une des revendications 1 à 6, l'élément piston (17, 117) étant déplacé en va-et-vient le long de la cavité intérieure de la partie de tête vers la première extrémité (11a, 111a) de la partie de tête (11, 111) en éjectant par cela du matériau de travail (50) fondu déposé sur une surface intérieure de la partie de tête tubulaire (11, 111).

10. Procédé de fonctionnement selon la revendication 9, l'élément piston (17, 117) étant déplacé en va-et-vient périodiquement avec une fréquence dans la gamme de 0,2 min⁻¹ à 0,0055 Hz.

11. Procédé de fonctionnement selon l'une des revendications 9 à 10, la durée d'un mouvement de va-et-vient de l'élément piston (17, 117) étant dans la gamme de 0,5 s à 10 s, de préférence dans la gamme de 0,5 s à 1,5 s.

12. Procédé de fonctionnement selon l'une des revendications 9 à 11, le mouvement de va-et-vient de l'élément piston (17, 117) étant amorti à la fin d'au moins une des phases de mouvement avant et arrière du mouvement de va-et-vient.

13. Procédé de fabrication d'une pièce de travail tubulaire perforée à partir d'un produit semi-fini (20) tubulaire en utilisant le système de découpe au laser selon l'une des revendications 7 à 8 et comprenant les étapes suivantes :
• disposer le block à faisceau (10, 110) à l'intérieur du produit semi-fini de façon que le produit (20) soit situé dans une zone de la première extrémité (11a, 111a) de la partie de tête tubulaire (11, 111),
• découper au laser le produit semi-fini en utilisant le couteau laser (30) et
• faire fonctionner le bloc à faisceau (10, 110) selon le procédé de fonctionnement selon l'une des revendications 9 à 11.

14. Procédé de fabrication selon la revendication 13, le mouvement de va-et-vient de l'élément piston du fonctionnement du block à faisceau étant effectué périodiquement pendant la découpe au laser du produit semi-fini (20).
